# EUROPÄISCHE PATENTSCHRIFT

(11) **EP 2 545 169 B1**
(45) Veröffentlichungstag und Bekanntmachung des Hinweises auf die Patenterteilung: **02.05.2018**
(21) Anmeldenummer: 11707420.3
(22) Anmeldetag: 10.03.2011
(51) Int. Cl.: C12N 9/54

(54) **LEISTUNGSVERBESSERTE PROTEASEVARIANTE**
PROTEASES WITH IMPROVED PERFORMANCE
PROTEASES AVEC DES EFFICIENCES AMELIORÉES

(30) Priorität: 11.03.2010 DE 102010002762
(43) Veröffentlichungstag der Anmeldung: 16.01.2013
(73) Patentinhaber: Henkel AG & Co. KGaA, 40589 Düsseldorf (DE)
(72) Erfinder: SIEGERT, Petra, 42781 Haan (DE); SCHWANEBERG, Ulrich, 4728 Kelmis-Hergenrath (BE); MARTINEZ MOYA, Ronny, 52064 Aachen (DE); MERKEL, Marion, 51145 Köln (DE); SPITZ, Astrid, 47441 Moers (DE); WIELAND, Susanne, 41541 Zons/Dormagen (DE); HELLMUTH, Hendrik, 40237 Düsseldorf (DE); MAURER, Karl-Heinz, 40699 Erkrath (DE)
(86) Internationale Anmeldenummer: PCT/EP2011/053607
(87) Internationale Veröffentlichungsnummer: WO 2011/110625

(56) Entgegenhaltungen:
- WO-A1-03/054184
- WO-A1-03/054185
- WO-A1-2008/086916
- WO-A2-2007/131657

## Beschreibung

Die Erfindung liegt auf dem Gebiet der Enzymtechnologie. Die Erfindung betrifft Insbesondere Proteasen sowie deren Herstellung, deren Aminosäuresequenz insbesondere Im Hinblick auf den Einsatz in Wasch- und Reinigungsmitteln verändert wurde, alle hinreichend ähnlichen Proteasen mit einer entsprechenden Veränderung und für sie codierende Nukleinsäuren. Die Erfindung betrifft ferner Verfahren und Verwendungen dieser Proteasen sowie sie enthaltende Mittel, insbesondere Wasch- und Reinigungsmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von Bacillus-Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Protease aus Bacillus lentus, insbesondere aus Bacillus lentus DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Reinigungsmitteln optimlert. Dazu gehören Punktmutagenese, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

In den internationalen Patentanmeldungen WO 03/054184, WO 2008/086919 und WO 2007/131657 werden alkalische Proteasen offenbart, auch für ihren Einsatz in Wasch- oder Reinigungsmitteln, die mit den hierin beschriebenen Proteasen Identitätswerte im Hinblick auf die Aminosäuresequenz von 91,1 %, 90,7% bzw. 91,1 % aufweisen.

In der internationalen Patentanmeldung WO 03/054185 sind alkalische Proteasen aus Bacillus gibsonii, (DSM 14391) offenbart, auch für ihren Einsatz in Wasch- oder Reinigungsmitteln. Dieser Stamm ist am 01.03.2001 gemäß dem Budapester Vertrag über die internationale Anerkennung der Hinterlegung von Mikroorganismen vom 28. April 1977 bei der Deutschen Sammlung von Mikroorganismen und Zellkulturen GmbH, Inhoffenstraße 7B, 38124 Braunschweig (http://www.dsmz.de) unter der Bezeichnung ID 01-192 und der Eingangsnummer DSM 14391 hinterlegt worden. Gegenüber den vorstehend genannten Proteasen weisen diese Proteasen erhebliche Unterschiede in der Aminosäuresequenz auf, so dass ein Identitätsvergleich der Aminosäuresequenzen Identitätswerte ergibt, die unter 80% Identität liegen. Für die alkalischen Proteasen aus Bacillus gibsonii (DSM 14391) sind bislang nur wenige für den Einsatz in Wasch- und Reinigungsmitteln optimierte Protease-Varianten im Stand der Technik bekannt.

Aufgabe der vorliegenden Erfindung ist es daher, eine Protease vom Typ der alkalischen Protease aus Bacillus gibsonii DSM 14391 bzw. eine hierzu hinreichend ähnliche Protease (bezogen auf die Sequenzidentität) weiterzuentwickeln und Protease-Varianten zu erhalten, die für ihren Einsatz in Wasch- oder Reinigungsmitteln geeignet und vorteilhafterweise verbessert sind.

Gegenstand der Erfindung ist eine Protease wie in Anspruch 1 definiert.

Ein weiterer Gegenstand der Erfindung ist ein Verfahren zur Herstellung einer solchen Protease wie in den Ansprüchen definiert.

Eine Protease im Sinne der vorliegenden Patentanmeldung umfasst daher sowohl die Protease als solche als auch eine mit einem erfindungsgemäßen Verfahren hergestellte Protease. Alle Ausführungen zur Protease beziehen sich daher sowohl auf die Protease als Stoff wie auch auf die entsprechenden Verfahren, insbesondere Herstellungsverfahren der Protease.

Als weitere Erfindungsgegenstände sind mit den erfindungsgemäßen Proteasen bzw. den Herstellungsverfahren für erfindungsgemäße Proteasen für diese Proteasen codierende Nukleinsäuren, erfindungsgemäße Proteasen oder Nukleinsäuren enthaltende nicht menschliche Wirtszellen sowie erfindungsgemäße Proteasen umfassende Mittel, insbesondere Wasch- und Reinigungsmittel, Wasch- und Reinigungsverfahren, und über erfindungsgemäßen Proteasen definierte Verwendungen verbunden.

Überraschenderweise wurde festgestellt, dass eine erfindungsgemäße Veränderung der Position 21 in einer Protease, die eine zu der in SEQ ID NO. 1 angegebenen Aminosäuresequenz zu mindestens 70% identische Aminosäuresequenz umfasst, eine verbesserte Leistung dieser veränderten Protease in Wasch- und Reinigungsmitteln bewirkt im Vergleich zu einer entsprechenden Protease, die diese Veränderungen nicht aufweist. Dies ist insbesondere deshalb überraschend, da sich die erfindungsgemäß veränderte Protease von weiteren im Stand der Technik etablierten Subtilisinen - wie beispielsweise Subtilisin 309, PB92, der alkalische Protease aus Bacillus lentus DSM 5483 oder BPN', etc. - deutlich unterscheidet. Beispielsweise ist eine Protease aufweisend SEQ ID NO. 1 zu Subtilisin 309 zu 78,4%, zu PB92 zu 78,1%, zur alkalischen Protease aus Bacillus lentus DSM 5483 zu 77,7% und zu BPN' zu 55,3% identisch, wobei SEQ ID NO. 1 die Sequenz der reifen Protease aus Bacillus gibsonii (DSM 14391) offenbart. Es war somit keinesfalls zu erwarten, dass für Proteasen des Typs alkalische Protease aus Bacillus gibsonii, (DSM 14391) für den Einsatz in Wasch- und Reinigungsmitteln leistungsverbesserte Protease-Varianten erhalten werden durch eine Veränderung an Position 21 in der Zählweise der alkalischen Proteasen aus Bacillus gibsonii, (DSM 14391), bezogen auf das reife Enzym gemäß SEQ ID NO. 1.

Bevorzugte Ausführungsformen erfindungsgemäßer Proteasen leisten beispielsweise einen so guten Beitrag zur Reinigungsleistung eines Wasch- oder Reinigungsmittels, welches die Protease enthält, der dem Beitrag von einem für diesen Zweck etablierten proteolytischen Enzym zur Reinigungsleistung des Mittels nahe kommt und ihn an unterschiedlichen Anschmutzungen sogar übertrifft. Erfindungsgemäße Proteasen ermöglichen folglich eine verbesserte Entfernung von mindestens einer, bevorzugt von mehreren protease-sensitiven Anschmutzungen auf Textilien und/oder harten Oberflächen, beispielsweise Geschirr. Besonders vorteilhafte Reinigungsleistungen zeigen bevorzugte Ausführungsformen erfindungsgemäßer Proteasen an Schokoladen- oder Kakao-haltigen Anschmutzungen. Folglich werden durch bevorzugte Ausführungsformen der vorliegenden Erfindung schmutzspezifische Proteasen bereitgestellt, deren Reinigungsleistung gezielt im Hinblick auf eine Anschmutzung oder auf mehrere Anschmutzungen ähnlichen Typs vorteilhaft ist. Folglich ist der Fleckenfokus bevorzugter Ausführungsformen erfindungsgemäßer Proteasen hinsichtlich Schokoladen- oder Kakao-haltigen Anschmutzungen verbessert.

Bevorzugte Ausführungsformen erfindungsgemäßer Proteasen erzielen solche vorteilhaften Reinigungsleistungen auch schon bei niedrigen Temperaturen zwischen 10°C und 40°C, zwischen 10°C und 30°C und zwischen 10°C und 25°C, beispielsweise bei 20°C.

Ferner verfügen bevorzugte Ausführungsformen erfindungsgemäßer Proteasen über eine besondere Stabilität gegenüber Tensiden und/oder Bleichmitteln und/oder gegenüber Temperatureinflüssen, insbesondere gegenüber hohen oder niedrigen Temperaturen und/oder gegenüber sauren oder alkalischen Bedingungen und/oder gegenüber pH-Wert-Änderungen und/oder gegenüber denaturierenden oder oxidierenden Agentien und/oder gegenüber proteolytischem Abbau und/oder gegenüber einer Veränderung der Redox-Verhältnisse.

Eine erfindungsgemäße Protease weist eine proteolytische Aktivität auf, das heißt, sie ist zur Hydrolyse von Peptidbindungen eines Polypeptids bzw. Proteins befähigt, insbesondere in einem Wasch- oder Reinigungsmittel. Eine erfindungsgemäße Protease ist daher ein Enzym, welches die Hydrolyse von Peptidbindungen katalysiert und dadurch in der Lage ist, Peptide oder Proteine zu spalten.

Eine erfindungsgemäße Protease ist auf Grund ihrer proteolytischen Aktivität und/oder ihren weiteren Eigenschaften, insbesondere betreffend ihre Stabilität gegenüber Tensiden und/oder Bleichmitteln und/oder ihr Temperaturprofil und/oder ihr pH-Profil, für die Anwendung in Wasch- und Reinigungsmitteln geeignet. Sie ermöglicht daher eine verbesserte Entfernung von mindestens einer, bevorzugt von mehreren protease-sensitiven Anschmutzungen auf Textilien und/oder harten Oberflächen, beispielsweise Geschirr. Besonders vorteilhafte Reinigungsleistungen zeigen erfindungsgemäße Proteasen beispielsweise an Gras und/oder Ei und/oder Schokoladenmilch bzw. Ruß und/oder Kakao und/oder Blut bzw. Milch enthaltenden Anschmutzungen, beispielsweise den Anschmutzungen
Gras auf Baumwolle: Produkt Nr. 164 erhältlich von der Firma Eidgenössische Material- und Prüfanstalt (EMPA) Testmaterialien AG, St. Gallen, Schweiz,
Voll-Ei/Pigment (Ganzei/Ruß) auf Baumwolle: Produkt Nr. 10N von der Firma wfk Testgewebe GmbH; Brüggen-Bracht, Deutschland,
Schokoladenmilch/Ruß auf Baumwolle: Produkt Nr. C-03 erhältlich von CFT (Center For Testmaterials) B.V. Vlaardingen, Niederlande,
Kakao auf Baumwolle: Produkt Nr. 112 erhältlich von der Firma Eidgenössische Material- und Prüfanstalt (EMPA) Testmaterialien AG, St. Gallen, Schweiz,
Blut-Milch/Tusche auf Baumwolle: Produkt Nr. C-05 erhältlich von CFT (Center For Testmaterials) B.V. Vlaardingen, Niederlande.
Ganz besonders vorteilhaft wirksam sind die erfindungsgemäßen Proteasen an Schokoladen- oder Kakao-haltige Anschmutzungen, beispielsweise den vorstehend genannten Anschmutzungen C-03 und/oder EMPA 112.

Weiter überraschend wurde festgestellt, dass solche vorteilhaften Reinigungsleistungen auch bei niedrigen Temperaturen zwischen 10°C und 40°C, zwischen 10°C und 30°C und zwischen 10°C und 25°C, beispielsweise bei 20°C, erzielt werden.

Unter Reinigungsleistung wird im Rahmen der Erfindung die Aufhellungsleistung an einer oder mehreren Anschmutzungen, insbesondere auf Wäsche oder Geschirr, verstanden. Im Rahmen der Erfindung weist sowohl das Wasch- oder Reinigungsmittel, welches die Protease umfasst bzw. die durch dieses Mittel gebildete Wasch- bzw. Reinigungsflotte, als auch die Protease selbst eine jeweilige Reinigungsleistung auf. Die Reinigungsleistung des Enzyms trägt somit zur Reinigungsleistung des Mittels bzw. der durch das Mittel gebildeten Wasch- bzw. Reinigungsflotte bei. Die Reinigungsleistung wird bevorzugt ermittelt wie weiter unten angegeben.

Eine bevorzugte Protease ist angegeben unter SEQ ID NO. 2, d.h. bevorzugt umfasst eine erfindungsgemäße Protease SEQ ID NO. 2.

Überraschenderweise wurde festgestellt, dass weitere alternative Möglichkeiten vorhanden sind, die vorhandene Aminosäure in Position 21 zu verändern, um eine Leistungsverbesserung der resultierenden Protease zu erhalten. Grundsätzlich ist es von Bedeutung, dass die Protease gegenüber SEQ ID NO. 1 an dieser Positionen überhaupt verändert ist, d.h. die an der dieser Position vorhandene Aminosäure durch eine andere proteinogene Aminosäure ersetzt ist, also durch Alanin oder Arginin oder Asparagin oder Asparaginsäure oder Cystein oder Glutamin oder Glutaminsäure oder Glycin oder Histidin oder Leucin oder Lysin oder Methionin oder Phenylalanin oder Prolin oder Serin oder Threonin oder Tryptophan oder Tyrosin oder insbesondere Valin. Da Valin an dieser Position besonders vorteilhaft ist, sind aus den vorstehenden Aminosäuren zu Valin konservative Aminosäuren bevorzugt, d.h. solche, die - sofern Valin durch eine solche Aminosäure ersetzt wird - nicht zu einer Änderung der Polarität oder Ladung führen, insbesondere Glycin, Alanin, Isoleucin, Leucin und Methionin.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. In der vorliegenden Patentanmeldung wurden alle Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

In einer weiteren Ausführungsform ist die Protease dadurch gekennzeichnet, dass ihre Reinigungsleistung mindestens derjenigen einer Protease entspricht, die eine Aminosäuresequenz umfasst, die der in SEQ ID NO. 1 angegebenen Aminosäuresequenz entspricht, und/oder mindestens derjenigen einer Protease entspricht, die eine Aminosäuresequenz umfasst, die der in SEQ ID NO. 2 angegebenen Aminosäuresequenz entspricht, und/oder mindestens derjenigen einer Protease gemäß SEQ ID NO. 3 entspricht, wobei die Reinigungsleistung in einem Waschsystem bestimmt wird, das ein Waschmittel in einer Dosierung zwischen 4,5 und 7,0 Gramm pro Liter Waschflotte sowie die Protease enthält, wobei die zu vergleichenden Proteasen aktivitätsgleich eingesetzt sind und die Reinigungsleistung gegenüber einer oder mehrerer der Anschmutzungen Blut-Milch/Tusche auf Baumwolle, Schokoladenmilch/Ruß auf Baumwolle, Erdnuss Öl-Pigment/Tusche auf Polyester/Baumwolle und Gras auf Baumwolle, insbesondere gegenüber einer oder mehrerer der Anschmutzungen
- Blut-Milch/Tusche auf Baumwolle: Produkt Nr. C-05 erhältlich von CFT (Center For Testmaterials) B.V. Vlaardingen, Niederlande
- Schokoladenmilch/Ruß auf Baumwolle: Produkt Nr. C-03 erhältlich von CFT (Center For Testmaterials) B.V. Vlaardingen, Niederlande
- Erdnuss Öl-Pigment/Tusche auf Polyester/Baumwolle: Produkt Nr. PC-10 erhältlich von CFT (Center For Testmaterials) B.V. Vlaardingen, Niederlande
- Gras auf Baumwolle: Produkt Nr. 164 erhältlich von der Firma Eidgenössische Material- und Prüfanstalt (EMPA) Testmaterialien AG, St. Gallen, Schweiz,
bestimmt wird durch Messung des Weißheitsgrades der gewaschenen Textilien, der Waschvorgang für mindestens 30 Minuten, optional 60 Minuten, bei einer Temperatur von 40°C erfolgt und das Wasser eine Wasserhärte zwischen 15,5 und 16,5° (deutsche Härte) aufweist.

Ein bevorzugtes flüssiges Waschmittel für ein solches Waschsystem ist wie folgt zusammengesetzt (alle Angaben in Gewichts-Prozent): 0,3-0,5% Xanthan, 0,2-0,4% Anti-Schaummittel, 6-7% Glycerin, 0,3-0,5% Ethanol, 4-7% FAEOS (Fettalkoholethersulfat), 24-28% nichtionische Tenside, 1 % Borsäure, 1-2% Natriumcitrat (Dihydrat), 2-4% Soda, 14-16% Kokosnuss-Fettsäuren, 0,5% HEDP (1-Hydroxyethan-(1,1-di-phosphonsäure)), 0-0,4% PVP (Polyvinylpyrrolidon), 0-0,05% optischer Aufheller, 0-0,001% Farbstoff, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 8 und pH 10,5, bevorzugt zwischen pH 8 und pH 9.

Ein bevorzugtes pulverförmiges Waschmittel für ein solches Waschsystem ist wie folgt zusammengesetzt (alle Angaben in Gewichts-Prozent): 10% lineares Alkylbenzolsulfonat (Natrium-Salz), 1,5% C12-C18-Fettalkoholsulfat (Natrium-Salz), 2,0% C12-C18-Fettalkohol mit 7 EO, 20% Natriumcarbonat, 6,5 % Natriumhydrogencarbonat, 4,0 % amorphes Natriumdisilikat, 17% Natriumcarbonat-peroxohydrat, 4,0% TAED, 3,0% Polyacrylat, 1,0% Carboxymethylcellulose, 1,0% Phosphonat, 25% Natriumsulfat, Rest: Schauminhibitoren, optischer Aufheller, Duftstoffe. Bevorzugt beträgt die Dosierung des pulverförmigen Waschmittels zwischen 5,5 und 7,0 Gramm pro Liter Waschflotte, beispielsweise 5,6, 5,9 oder 6,7 Gramm pro Liter Waschflotte. Bevorzugt wird gewaschen in einem pH-Wertebereich zwischen pH 9 und pH 11.

Im Rahmen der Erfindung erfolgt die Bestimmung die Reinigungsleistung bei 40°C unter Verwendung eines festen Waschmittels wie vorstehend angegeben.

Der Weißheitsgrad, d.h. die Aufhellung der Anschmutzungen, als Maß für die Reinigungsleistung wird bevorzugt mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann. Verfahren zur Bestimmung der Proteaseaktivität sind dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Bluret-Verfahren (A. G. Gornall, C. S. Bardawill und M.M. David, J. Biol. Chem., 177 (1948), S. 751-766) bestimmt werden. Die Bestimmung der Aktivproteinkonzentration kann diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913) erfolgen.

Die Proteaseaktivität wird üblicherweise in Protease-Einheiten (PE) angegeben. Geeignete Proteaseaktivitäten betragen beispielsweise 2,25, 5 oder 10 PE pro ml Waschflotte. Die Proteaseaktivität ist jedoch nicht gleich Null.

Zusätzlich zu den vorstehend erläuterten Aminosäureveränderungen können die hierin offenbarten Proteasen weitere Aminosäureveränderungen, insbesondere Aminosäure-Substitutionen, -Insertionenen oder -Deletionen, aufweisen. Solche Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden.

Das Ziel Ist es, in die bekannten Moleküle gezielte Mutationen wie Substitutionen, Insertionen oder Deletlonen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease, zum Beispiel hinsichtlich ihrer Stabilität gegenüber Tensiden und/oder Bleichmitteln und/oder anderen Komponenten, kann daher im Rahmen der Erfindung zusätzlich weiterentwickelt sein.

Für die Beschreibung von Substitutionen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletlonen ist die fehlende Aminosäure durch ein Symbol, beispielsweise einen Stern oder einen Strich, ersetzt. Beispielsweise beschreibt A95G die Substitution von Alanin an Position 95 durch Glycin, A95AG die Insertion von Glycin nach der Aminosäure Alanin an Position 95 und A95* die Deletion von Alanin an Position 95. Diese Nomenklatur ist dem Fachmann auf dem Gebiet der Enzymtechnologie bekannt.

Ein weiterer Gegenstand der vorliegenden Offenbarung ist daher eine Protease, die dadurch gekennzeichnet ist, dass sie aus einer Protease wie vorstehend beschrieben als Ausgangsmolekül erhaltbar ist durch ein- oder mehrfache konservative Aminosäuresubstitution, wobei die Protease in der Zählung gemäß SEQ ID NO. 1 die Aminosäuresubstitution I21V aufweist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Alternativ oder ergänzend ist die Protease dadurch gekennzeichnet, dass sie aus einer erfindungsgemäßen Protease als Ausgangsmolekül erhaltbar ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 266 oder 267 zusammenhängenden Aminosäuren mit dem Ausgangsmolekül übereinstimmt, wobei die im Ausgangsmolekül enthaltene Aminosäuresubstitution 121V noch vorhanden ist.

So ist es beispielsweise möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms. Auch Substitionen können vorteilhafte Wirkungen zeigen. Sowohl einzelne wie auch mehrere zusammenhängende Aminosäuren können gegen andere Aminosäuren ausgetauscht werden.

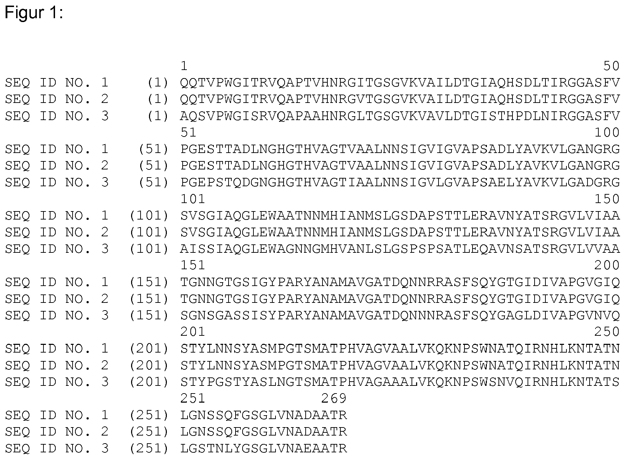

Alternativ oder ergänzend ist die Protease dadurch gekennzeichnet, dass sie aus einer erfindungsgemäßen Protease als Ausgangsmolekül erhaltbar ist durch eine oder mehrere Aminosäuresubstitutionen in Positionen, die den Positionen 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 und 268 der Protease aus Bacillus lentus gemäß SEQ ID NO. 3 in einem Alignment zugeordnet sind, wobei die Protease in der Zählung gemäß SEQ ID NO. 1 die Aminosäuresubstitution I21V aufweist. Die weiteren Aminosäurepositionen werden hierbei durch ein Alignment der Aminosäuresequenz einer erfindungsgemäßen Protease mit der Aminosäuresequenz der Protease aus Bacillus lentus, wie sie in SEQ ID NO. 3 angegeben ist, definiert. Ein solches Alignment ist in Figur 1 angegeben. Da die Protease aus Bacillus lentus im Stand der Technik ein wichtiges Referenzmolekül zur Beschreibung neuer Proteasen und von Aminosäureveränderungen darstellt und die hier beschriebenen neuen Proteasen und somit auch ihre Sequenz bislang unbekannt sind, ist es vorteilhaft, in der Zuordnung der Aminosäurepositionen auf die Protease aus Bacillus lentus (SEQ ID NO. 3) Bezug zu nehmen. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere Zahl von Aminosäurenresten umfasst als die Protease aus Bacillus lentus gemäß SEQ ID NO. 3. Ausgehend von den genannten Positionen in der Aminosäuresequenz der Protease aus Bacillus lentus sind die Veränderungspositionen in einer erfindungsgemäßen Protease diejenigen, die eben diesen Positionen in einem Alignment, beispielsweise gemäß Figur 1, zugeordnet sind.

Vorteilhafte Positionen für Sequenzveränderungen, insbesondere Substitutionen, der Protease aus Bacillus lentus, die übertragen auf homologe Positionen der erfindungsgemäßen Proteasen bevorzugt von Bedeutung sind und der Protease vorteilhafte funktionelle Eigenschaften verleihen, sind demnach die Positionen 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 und 268, zuzuordnen in einem Alignment mit SEQ ID NO. 3 und damit in der Zählung gemäß SEQ ID NO. 3. In den genannten Positionen liegen in dem Wildtypmolekül der Protease aus Bacillus lentus folgende Aminosäurereste: S3, V4, S36, N42, A47, T56, G61, T69, E87, A96, R99, A101, 1102, S104, N114, H118, A120, S130, S139, T141, S142, S154, S157, A188, V193, V199, G205, L211, A224, K229, S236, N237, N242, H243, N255 beziehungsweise T268.

Vorteilhaft sind insbesondere beispielsweise Substitutionen 3T, 4I, 61A, 99G, 99A, 99S, 99E, 154D, 154E, 211D, 211G und 211E, sofern die entsprechend homologen Positionen in einer erfindungsgemäßen Protease nicht schon natürlicherweise von einer dieser bevorzugten Aminosäuren eingenommen werden. Die Austausche 3T und 4I führen über einen Stabilisierungseffekt auf das Molekül zu einer Verbesserung der Reinigungsleistung der Protease und damit zu einer verbesserten Reinigungsleistung eines Wasch- oder Reinigungsmittels, das die Protease enthält.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht beispielsweise ein Aminosäureaustausch in einer bestimmten Position der Protease aus Bacillus lentus gemäß SEQ ID NO. 3 mit einer Veränderung eines enzymatischen Parameters einher, beispielsweise mit der Erhöhung des K_{M}-Wertes, und wird eine entsprechende Veränderung des enzymatischen Parameters, beispielsweise also ebenfalls eine Erhöhung des K_{M}-Wertes, in einer erfindungsgemäßen Protease-Variante beobachtet, deren Aminosäureaustausch durch dieselbe eingeführte Aminosäure erreicht wurde, so ist hierin eine Bestätigung dieses Erfindungsaspekts zu sehen.

Alle genannten Sachverhalte sind auch auf die hierin beschriebenen Verfahren zur Herstellung einer Protease anwendbar. Demnach umfasst ein solches Verfahren ferner einen oder mehreren der folgenden Verfahrensschritte:
(a) Einbringen einer ein- oder mehrfachen konservativen Aminosäuresubstitution, wobei die Protease in der Zählung gemäß SEQ ID NO. 1 die Aminosäuresubstitution I21V aufweist;
b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 266 oder 267 zusammenhängenden Aminosäuren mit dem Ausgangsmolekül übereinstimmt, wobei die im Ausgangsmolekül enthaltene Aminosäuresubstitution I21V noch vorhanden ist;
(c) Einbringen einer ein- oder mehrfachen Aminosäuresubstitution in eine oder mehrere der Positionen, die den Positionen 3, 4, 36, 42, 47, 56, 61, 69, 87, 96, 99, 101, 102, 104, 114, 118, 120, 130, 139, 141, 142, 154, 157, 188, 193, 199, 205, 211, 224, 229, 236, 237, 242, 243, 255 und 268 der Protease aus Bacillus lentus gemäß SEQ ID NO. 3 in einem Alignment zugeordnet sind, wobei die Protease in der Zählung gemäß SEQ ID NO. 1 die Aminosäuresubstitution I21V aufweist.

Sämtliche Ausführungen gelten auch für die erfindungsgemäßen Verfahren.

Die Protease beziehungsweise die mit einem erfindungsgemäßen Verfahren hergestellte Protease weist die Aminosäuresubstitution I21V auf.

Ein weiterer Gegenstand der vorliegenden Offenbarung ist eine Protease wie vorstehend beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise in vivo durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch in vitro durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Proteasen mit Protease-Inhibitoren ist vorteilhaft.

Betreffend alle vorstehend beschriebenen Proteasen bzw. Proteasevarianten und/oder Derivate sind diejenigen besonders bevorzugt, deren Aktivität mindestens derjenigen der Protease gemäß SEQ ID NO. 1 und/oder SEQ ID NO. 2 und/oder SEQ ID NO. 3 entspricht, und/oder deren Reinigungsleistung mindestens derjenigen der Protease gemäß SEQ ID NO. 1 und/oder SEQ ID NO. 2 und/oder SEQ ID NO. 3 entspricht, wobei die Reinigungsleistung in einem Waschsystem bestimmt wird wie vorstehend beschrieben.

Ein weiterer Gegenstand der Erfindung ist eine Nukleinsäure, die für eine erfindungsgemäße Protease codiert, sowie ein Vektor enthaltend eine solche Nukleinsäure, insbesondere ein Klonierungsvektor oder ein Expressionsvektor.

Hierbei kann es sich DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesen Erfindungsgegenstand mit eingeschlossen, die eine der vorstehend beschriebenen Proteasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Usage. Unter Codon-Usage wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Ein Beispiel einer besonders bevorzugten Nukleinsäure ist in SEQ ID NO. 4 angegeben.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom bzw. chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure bzw. ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile bzw. -Fragmente einer erfindungsgemäßen Nukleinsäure derart in eine Wirtszelle eingebracht werden, dass die dann resultierende Wirtszelle eine erfindungsgemäße Nukleinsäure bzw. einen erfindungsgemäßen Vektor enthält. Dieses Vorgehen eignet sich besonders dann, wenn die Wirtszelle bereits einen oder mehrere Bestandteile einer erfindungsgemäßen Nukleinsäure bzw. eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Protease funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryontische oder bakterielle Zellen. Bakterien zeichnen sich durch kurze Generationszeiten und geringe Ansprüche an die Kultivierungsbedingungen aus. Dadurch können kostengünstige Kultivierungsverfahren bzw. Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise Escherichia coli wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der Actinomycetales besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Usage verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung Bacillus, anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von Escherichia, Klebsiella, Bacillus, Staphylococcus, Corynebakterium, Arthrobacter, Streptomyces, Stenotrophomonas und Pseudomonas, weiter bevorzugt eines, das ausgewählt ist aus der Gruppe von Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia.

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryontischen Zellen sind eukaryontische Zellen in der Lage, das gebildete Protein posttranslational zu modifizieren. Beispiele dafür sind Pilze wie Actinomyceten oder Hefen wie Saccharomyces oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen oder Lipasen, kann vorteilhaft sein. Ferner können sich beispielsweise thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Proteasen herzustellen. Ein weiterer Gegenstand der Erfindung ist daher ein Verfahren zur Herstellung einer Protease umfassend
a) Kultivieren einer erfindungsgemäßen Wirtszelle
b) Isolieren der Protease aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Protease. Alle Fermentationsverfahren, die auf einem entsprechenden Verfahren zur Herstellung einer erfindungsgemäßen Protease beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse bzw. Trockenmasse und/oder vor allem der Aktivität der interessierenden Protease erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern bzw. -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Proteasen herzustellen.

Ein weiterer Gegenstand der Erfindung ist ein Mittel, das dadurch gekennzeichnet ist, dass es eine erfindungsgemäße Protease wie vorstehend beschrieben enthält. Bevorzugt ist das Mittel ein Wasch- oder Reinigungsmittel. Da erfindungsgemäße Proteasen vorteilhafte Reinigungsleistungen insbesondere an Schokoladen- oder Kakao-haltigen Anschmutzungen aufweisen, sind die Mittel insbesondere zur Entfernung von solchen Anschmutzungen geeignet und vorteilhaft.

Zu diesem Erfindungsgegenstand zählen alle denkbaren Wasch- bzw. Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als insbesondere pulverförmige Feststoffe, in nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder, Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Protease mit einem oder mehreren weiteren Inhaltsstoff(en) des Mittels ist vorteilhaft, da ein solches Mittel eine verbesserte Reinigungsleistung durch sich ergebende Synergismen aufweist. Insbesondere durch die Kombination einer erfindungsgemäßen Protease mit einem Tensid und/oder einem Builder (Gerüststoff) und/oder einer Persauerstoffverbindung und/oder einem Bleichaktivator kann ein solcher Synergismus erreicht werden.

Vorteilhafte Inhaltsstoffe erfindungsgemäßer Mittel sind offenbart in der internationalen Patentanmeldung WO2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Ein erfindungsgemäßes Mittel enthält die Protease vorteilhafterweise in einer Menge von 2µg bis 20mg, vorzugsweise von 5µg bis 17,5mg, besonders bevorzugt von 20µg bis 15mg und ganz besonders bevorzugt von 50µg bis 10mg pro g des Mittels. Ferner kann die in dem Mittel enthaltene Protease, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In weiteren Ausführungsformen der Erfindung ist das Mittel dadurch gekennzeichnet, dass es
(a) in fester Form vorliegt, insbesondere als rieselfähiges Pulver mit einem Schüttgewicht von 300g/l bis 1200g/l, insbesondere 500g/l bis 900g/l, oder
(b) in pastöser oder in flüssiger Form vorliegt, und/oder
(c) als Einkomponentensystem vorliegt, oder
(d) in mehrere Komponenten aufgeteilt ist.

Diese Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300g/l bis 1200g/l, insbesondere 500g/l bis 900g/l oder 600g/l bis 850g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Erfindungsgemäße Wasch- oder Reinigungsmittel können ausschließlich eine erfindungsgemäße Protease enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder eine Lipase, sowie deren Gemische. Weitere Enzyme sind in dem Mittel vorteilhafterweise jeweils in einer Menge von 1 x 10⁻⁸ bis 5 Gewichts-Prozent bezogen auf aktives Protein enthalten. Zunehmend bevorzugt ist jedes weitere Enzym in einer Menge von 1 x 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solches Synergismus vor zwischen der erfindungsgemäß enthaltenen Protease und einem weiteren Enzym eines erfindungsgemäßen Mittels, darunter insbesondere zwischen der genannten Protease und der Amylase und/oder einer Lipase und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet ist, oder dass in mindestens einem Verfahrensschritt eine erfindungsgemäße Protease oder eine nach einem erfindungsgemäßen Verfahren erhaltene Protease katalytisch aktiv ist, insbesondere derart, dass die Protease in einer Menge von 40µg bis 4g, vorzugsweise von 50µg bis 3g, besonders bevorzugt von 100µg bis 2g und ganz besonders bevorzugt von 200µg bis 1g pro Anwendung eingesetzt ist.

Hierunter fallen sowohl manuelle als auch maschinelle Verfahren, wobei maschinelle Verfahren bevorzugt sind. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung bzw. Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels bereichert werden und stellen dann Ausführungsformen der vorliegenden Erfindung dar. Alle Sachverhalte, Gegenstände und Ausführungsformen, die für erfindungsgemäße Proteasen bzw. sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da erfindungsgemäße Proteasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einziger Schritt eines solchen Verfahrens darin bestehen, dass gewünschtenfalls als einzige reinigungsaktive Komponente eine solche Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Ein weiterer Erfindungsgegenstand ist die Verwendung eines erfindungsgemäßen Mittels zur Reinigung von Textilien oder von harten Oberflächen oder einer erfindungsgemäßen Protease oder einer nach einem erfindungsgemäßen Verfahren erhaltenen Protease zur Reinigung von Textilien oder von harten Oberflächen, insbesondere derart, dass die Protease in einer Menge von 40µg bis 4g, vorzugsweise von 50µg bis 3g, besonders bevorzugt von 100µg bis 2g und ganz besonders bevorzugt von 200µg bis 1g pro Anwendung eingesetzt ist.

Alle Sachverhalte, Gegenstände und Ausführungsformen, die für erfindungsgemäße Proteasen bzw. sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

### Beispiel 1

Ausgehend von einer Protease, die eine Aminosäuresequenz gemäß SEQ ID NO. 1 aufwies, wurde eine erfindungsgemäße Protease-Variante durch ortsgerichtete ("site-directed") Mutagenese in der für die Protease codierenden Nukleinsäure hergestellt mittels der SeSaM-Methode (Wong, T. S. et al. (2004): Sequence saturation mutagenesis (SeSaM): a novel method for directed evolution. Nucleic Acids Res. 32, 26ff) hergestellt. Hierbei wurde das Codon für die Aminosäureposition 21 von ATA nach GTA geändert, so dass bezogen auf die Aminosäuresequenz ein Austausch von Isoleucin (I) gegen Valin (V) erfolgte. Die Expression der Protease-Variante erfolgte in fachüblicher Art- und Weise durch Transformation von Bacillus subtilis DB 104 (Kawamura und Doi (1984), J. Bacteriol., Band 160 (1), S. 442-444) mit einem entsprechenden Expressionsvektor und nachfolgender Kultur der die Protease-Variante exprimierenden Transformanden.

### Beispiel 2: Ermittlung der Reinigungsleistung bei Einsatz in einem handelsüblichen pulverförmigen Waschmittel

Für dieses Beispiel wurden standardisiert verschmutze Textilien eingesetzt. Es wurden folgende Anschmutzung und Textilien verwendet:
A: Gras auf Baumwolle: Produkt Nr. 164 erhältlich von der Firma Eidgenössische Material- und Prüfanstalt (EMPA) Testmaterialien AG, St. Gallen, Schweiz
B: Voll-Ei/Pigment (Ganzei/Ruß) auf Baumwolle: Produkt Nr. 10N von der Firma wfk Testgewebe GmbH; Brüggen-Bracht, Deutschland;
C: Schokoladenmilch/Ruß auf Baumwolle: Produkt Nr. C-03 erhältlich von CFT (Center For Testmaterials) B.V. Vlaardingen, Niederlande
D: Kakao auf Baumwolle: Produkt Nr. 112 erhältlich von der Firma Eidgenössische Material- und Prüfanstalt (EMPA) Testmaterialien AG, St. Gallen, Schweiz
E: Blut-Milch/Tusche auf Baumwolle: Produkt Nr. C-05 erhältlich von CFT (Center For Testmaterials) B.V. Vlaardingen, Niederlande

Mit diesem Testmaterial wurden verschiedene Waschmittelrezepturen auf ihre Reinigungsleistung hin untersucht. Dafür wurden die Ansätze für 60 Minuten bei einer Temperatur von 40°C gewaschen. Die Dosierung lag bei 5,9g des Waschmittels pro Liter Waschflotte. Es wurde mit Stadtwasser mit einer Wasserhärte von 16° deutscher Härte gewaschen.

Als Kontroll-Waschmittel diente eine Waschmittel-Basis-Rezeptur folgender Zusammensetzung (alle Angaben in Gewichts-Prozent): 10% lineares Alkylbenzolsulfonat (Natrium-Salz), 1,5% C12-C18-Fettalkoholsulfat (Natrium-Salz), 2,0% C12-C18-Fettalkohol mit 7 EO, 20% Natriumcarbonat, 6,5% Natriumhydrogencarbonat, 4,0% amorphes Natriumdisilikat, 17% Natriumcarbonatperoxohydrat, 4,0% TAED, 3,0% Polyacrylat, 1,0% Carboxymethylcellulose, 1,0% Phosphonat, 25% Natriumsulfat, Rest: Schauminhibitoren, optischer Aufheller, Duftstoffe. Die Waschmittel-Basis-Rezeptur wurde für die verschiedenen Versuchsreihen aktivitätsgleich (5 PE/ml Endkonzentration) mit folgenden Proteasen versetzt: erfindungsgemäße Protease-Variante mit einer Aminosäuresequenz gemäß SEQ ID NO. 1 I21V (demnach entsprechend SEQ ID NO. 2, nachfolgend bezeichnet als Ansatz 1) und eine entsprechende Kontrollprotease gemäß SEQ ID NO. 1, die die Aminosäuresubstitution I21V nicht aufweist (Ansatz 2).

Nach dem Waschen wurde der Weißheitsgrad der gewaschenen Textilien gemessen. Die Messung erfolgte an einem Spektrometer Minolta CM508d (Lichtart D65, 10°). Das Gerät wurde zuvor mit einem mitgelieferten Weißstandard kalibriert. Die erhaltenen Ergebnisse sind die Differenzremissionen zwischen einem Waschvorgang mit einem Waschmittel enthaltend eine Protease und einem parallel durchgeführten Kontrollwaschgang mit einem Waschmittel ohne Protease. STABW bezeichnet die Standardabweichung bei parallel durchgeführten Versuchsansätzen. Die Ergebnisse sind in der nachfolgenden Tabelle 1 zusammengestellt und erlauben einen unmittelbaren Rückschluss auf den Beitrag des jeweils enthaltenen Enzyms zur Reinigungsleistung des verwendeten Mittels.

**Tabelle 1: Waschergebnisse mit einem pulverförmigen Waschmittel bei 40°C**

| Anschmutzung | Ansatz 1 | STABW | Ansatz 2 | STABW |
|---|---|---|---|---|
| A | 4,8 | 0,7 | 3,0 | 0,2 |
| B | 7,4 | 0,7 | 6,9 | 0,4 |
| C | 11,1 | 0,9 | 8,6 | 0,6 |
| D | 5,6 | 0,2 | 2,0 | 0,5 |
| E | 13,3 | 1,6 | 11,4 | 0,6 |

Es wird deutlich, dass die erfindungsgemäße Protease eine bessere Reinigungsleistung zeigt im Vergleich zur Kontrolle. Ferner zeigt die erfindungsgemäße Protease insbesondere an Schokoladen- oder Kakao-haltigen Anschmutzungen sehr gute Reinigungsleistungen.

### Beschreibung der Figur:

Figur 1:
Sequenzvergleich (Alignment) der Sequenzen gemäß SEQ ID NO. 1, SEQ ID NO. 2 und SEQ ID NO. 3, erstellt mit dem Programm Vector NTI® Suite 10.3 (Invitrogen Corporation, 1600 Faraday Avenue, Carlsbad, Kalifornien, USA) unter Standardparametern.

## Patentansprüche

1. Protease mit der Aminosäuresequenz wie unter SEQ ID NO. 2 angegeben.

2. Nukleinsäure codierend für eine Protease nach Anspruch 1.

3. Vektor enthaltend eine Nukleinsäure nach Anspruch 2, insbesondere ein Klonierungsvektor oder ein Expressionsvektor.

4. Nicht menschliche Wirtszelle, die eine Nukleinsäure nach Anspruch 2 oder einen Vektor nach Anspruch 3 beinhaltet, oder die eine Protease nach Anspruch 1 beinhaltet, insbesondere eine, die die Protease in das die Wirtszelle umgebende Medium sezerniert.

5. Wirtszelle nach Anspruch 4, **dadurch gekennzeichnet, dass** sie einen Zellkern besitzt, oder dass sie ein Bakterium ist, bevorzugt eines, das ausgewählt ist aus der Gruppe der Gattungen von Escherichia, Klebsiella, Bacillus, Staphylococcus, Corynebakterium, Arthrobacter, Streptomyces, Stenotrophomonas und Pseudomonas, weiter bevorzugt eines, das ausgewählt ist aus der Gruppe von Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus carnosus, Corynebacterium glutamicum, Arthrobacter oxidans, Streptomyces lividans, Streptomyces coelicolor und Stenotrophomonas maltophilia.

6. Verfahren zur Herstellung einer Protease umfassend
a) Kultivieren einer Wirtszelle gemäß einem der Ansprüche 4 oder 5;
b) Isolieren der Protease aus dem Kulturmedium oder aus der Wirtszelle.

7. Mittel, insbesondere ein Wasch- oder Reinigungsmittel, **dadurch gekennzeichnet, dass** es mindestens eine Protease nach Anspruch 1 enthält, insbesondere in einer Menge von 2µg bis 20mg, vorzugsweise von 5µg bis 17,5mg, besonders bevorzugt von 20µg bis 15mg und ganz besonders bevorzugt von 50µg bis 10mg pro g des Mittels,
und/oder dass es mindestens eine Protease nach Anspruch 1 enthält und die Protease in dem Mittel mit einer bei Raumtemperatur oder bei Abwesenheit von Wasser für die Protease undurchlässigen Substanz umhüllt ist.

8. Mittel nach Anspruch 7, **dadurch gekennzeichnet, dass** es
(a) in fester Form vorliegt, insbesondere als rieselfähiges Pulver mit einem Schüttgewicht von 300g/l bis 1200g/l, insbesondere 500g/l bis 900g/l, oder
(b) in pastöser oder in flüssiger Form vorliegt, und/oder
(c) als Einkomponentensystem vorliegt, oder
(d) in mehrere Komponenten aufgeteilt ist.

9. Verfahren zur Reinigung von Textilien oder harten Oberflächen, **dadurch gekennzeichnet, dass** in mindestens einem Verfahrensschritt ein Mittel nach einem der Ansprüche 7 oder 8 angewendet ist, oder dass in mindestens einem Verfahrensschritt eine Protease nach Anspruch 1 katalytisch aktiv ist, insbesondere derart, dass die Protease in einer Menge von 40µg bis 4g, vorzugsweise von 50µg bis 3g, besonders bevorzugt von 100µg bis 2g und ganz besonders bevorzugt von 200µg bis 1g pro Anwendung eingesetzt ist.

10. Verwendung eines Mittels nach einem der Ansprüche 7 oder 8 zur Reinigung von Textilien oder von harten Oberflächen oder einer Protease nach Anspruch 1 zur Reinigung von Textilien oder von harten Oberflächen, insbesondere derart, dass die Protease in einer Menge von 40µg bis 4g, vorzugsweise von 50µg bis 3g, besonders bevorzugt von 100µg bis 2g und ganz besonders bevorzugt von 200µg bis 1g pro Anwendung eingesetzt ist.

## Claims

1. A protease having the amino acid sequence as indicated by SEQ ID NO. 2.

2. A nucleic acid coding for a protease according to claim 1.

3. A vector containing a nucleic acid according to claim 2, in particular a cloning vector or an expression vector.

4. A non-human host cell containing a nucleic acid according to claim 2 or a vector according to claim 3, or containing a protease according to claim 1, in particular a non-human host cell which secretes the protease into the medium surrounding the host cell.

5. The host cell according to claim 4, **characterized in that** it has a cell nucleus, or **in that** it is a bacterium, preferably one selected from the group containing the genera of Escherichia, Klebsiella, Bacillus, Staphylococcus, Corynebacterium, Arthrobacter, Streptomyces, Stenotrophomonas and Pseudomonas, more preferably one selected from the group containing Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus carnosus, Corynebacterium glutamicum, Arthrobacter oxidans, Streptomyces lividans, Streptomyces coelicolor and Stenotrophomonas maltophilia.

6. A method for preparing a protease, comprising
a) cultivating a host cell according to one of claims 4 or 5;
b) isolating the protease from the culture medium or from the host cell.

7. An agent, in particular a washing or cleaning agent, **characterized in that** it contains at least one protease according to claim 1, in particular in an amount of from 2 µg to 20 mg, preferably 5 µg to 17.5 mg, particularly preferably 20 µg to 15 mg and very particularly preferably 50 µg to 10 mg per g of the agent,
and/or **in that** it contains at least one protease according to claim 1, and the protease is encapsulated in the agent by a substance impermeable to the protease at room temperature or in the absence of water.

8. The agent according to claim 7, **characterized in that**
(a) it is in solid form, in particular as a flowable powder having a bulk density of from 300 g/l to 1200 g/l, in particular 500g/l to 900 g/l, or
(b) is present in paste or in liquid form, and/or
(c) is present as a single-component system, or
(d) is divided into a plurality of components.

9. A method for cleaning textiles or hard surfaces, **characterized in that** an agent according to one of claims 7 or 8 is applied in at least one method step, or **in that** a protease according to claim 1 is catalytically active in at least one method step, in particular such that the protease is used in an amount of from 40 µg to 4 g per application, preferably 50 µg to 3 g, particularly preferably 100 µg to 2 g and very particularly preferably 200 µg to 1 g.

10. The use of an agent according to one of claims 7 or 8 for cleaning textiles or hard surfaces, or a protease according to claim 1 for cleaning textiles or hard surfaces, in particular such that the protease is used in an amount of from 40 µg to 4 g per application, preferably 50 µg to 3 g, particularly preferably 100 µg to 2 g and very particularly preferably 200 µg to 1 g.

## Revendications

1. Protéase ayant la séquence d'acides aminés comme indiqué en SEQ ID n°2.

2. Acide nucléique codant une protéase selon la revendication 1.

3. Vecteur contenant un acide nucléique selon la revendication 2, en particulier un vecteur de clonage ou un vecteur d'expression.

4. Cellule hôte non-humaine, contenant un acide nucléique selon la revendication 2 ou un vecteur selon la revendication 3 ou une protéase selon la revendication 1, en particulier qui sécrète la protéase dans le milieu entourant la cellule hôte.

5. Cellule hôte selon la revendication 4, **caractérisée en ce qu'**elle possède un noyau cellulaire ou **en ce qu'**elle est une bactérie, de préférence une choisie dans le groupe des genres Escherichia, Klebsiella, Bacillus, Staphylococcus, Corynebacterium, Arthrobacter, Streptomyces, Stenotrophomonas et Pseudomonas, plus préférentiellement une choisie dans le groupe de Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus pumilus, Staphylococcus carnosus, Corynebacterium glutamicum, Arthrobacter oxidans, Streptomyces lividans, Streptomyces coelicolor et Stenotrophomonas maltophilia.

6. Procédé de production d'une protéase comprenant :
a) la culture d'une cellule hôte selon l'une des revendications 4 ou 5 ;
b) l'isolement de la protéase du milieu de culture ou de la cellule hôte.

7. Agent, en particulier agent de lavage ou de nettoyage, **caractérisé en ce qu'**il contient au moins une protéase selon la revendication 1, en particulier dans une quantité de 2 µg à 20 mg, de préférence de 5 µg à 17,5 mg, de manière particulièrement préférée de 20 µg à 15 mg et de manière tout particulièrement préférée de 50 µg à 10 mg par g d'agent,
et/ou **en ce qu'**il contient au moins une protéase selon la revendication 1 et **en ce que** la protéase est enveloppée dans l'agent par une substance qui ne laisse pas passer la protéase à la température ambiante ou en l'absence d'eau.

8. Agent selon la revendication 7, **caractérisé en ce que** :
(a) il se présente sous forme solide, en particulier sous forme de poudre fluide ayant une densité apparente de 300 g/l à 1200 g/l, en particulier de 500 g/l à 900 g/l, ou
(b) il se présente sous forme de pâte ou sous forme liquide, et/ou
(c) il se présente sous forme d'un système mono-composant, ou
(d) il est divisé en plusieurs composants.

9. Procédé de nettoyage de textiles ou de surfaces dures, **caractérisé en ce qu'**un agent selon la revendication 7 ou 8 est utilisé dans au moins une étape du procédé, ou **en ce qu'**une protéase selon la revendication 1 est catalytiquement active dans au moins une étape du procédé, en particulier de sorte que la protéase soit utilisée dans une quantité de 40 µg à 4 g, de préférence de 50 µg à 3 g, de manière particulièrement préférée de 100 µg à 2 g et de manière tout particulièrement préférée de 200 µg à 1 g par utilisation.

10. Utilisation d'un agent selon une des revendications 7 ou 8 pour nettoyer des textiles ou des surfaces dures ou d'une protéase selon la revendication 1 pour nettoyer des textiles ou des surfaces dures, en particulier de sorte que la protéase soit utilisée dans une quantité de 40 µg à 4 g, de préférence de 50 µg à 3 g, de manière particulièrement préférée de 100 µg à 2 g et de manière tout particulièrement préférée de 200 µg à 1 g par utilisation.


### SEQUENCE LISTING

<110> Henkel AG & Co. KGaA
<120> Leistungsverbesserte Proteasevariante
<130> PT018262 (H 08847 PCT)
<150> DE 102010002762
   <151> 2010-03-11
<160> 4
<170> PatentIn version 3.3
<210> 1
   <211> 269
   <212> PRT
   <213> Bacillus sp.
<400> 1
   Gln Gln Thr Val Pro Trp Gly Ile Thr Arg Val Gln Ala Pro Thr Val
   1               5                   10                  15
   His Asn Arg Gly Ile Thr Gly Ser Gly Val Lys Val Ala Ile Leu Asp
               20                  25                  30
   Thr Gly Ile Ala Gln His Ser Asp Leu Thr Ile Arg Gly Gly Ala Ser
           35                  40                  45
   Phe Val Pro Gly Glu Ser Thr Thr Ala Asp Leu Asn Gly His Gly Thr
       50                  55                  60
   His Val Ala Gly Thr Val Ala Ala Leu Asn Asn Ser Ile Gly Val Ile
   65                  70                  75                  80
   Gly Val Ala Pro Ser Ala Asp Leu Tyr Ala Val Lys Val Leu Gly Ala
                   85                  90                  95
   Asn Gly Arg Gly Ser Val Ser Gly Ile Ala Gln Gly Leu Glu Trp Ala
               100                 105                 110
   Ala Thr Asn Asn Met His Ile Ala Asn Met Ser Leu Gly Ser Asp Ala
           115                 120                 125
   Pro Ser Thr Thr Leu Glu Arg Ala Val Asn Tyr Ala Thr Ser Arg Gly
       130                 135                 140
   Val Leu Val Ile Ala Ala Thr Gly Asn Asn Gly Thr Gly Ser Ile Gly
   145                 150                 155                 160
   Tyr Pro Ala Arg Tyr Ala Asn Ala Met Ala Val Gly Ala Thr Asp Gln
                   165                 170                 175
   Asn Asn Arg Arg Ala Ser Phe Ser Gln Tyr Gly Thr Gly Ile Asp Ile
               180                 185                 190
   Val Ala Pro Gly Val Gly Ile Gln Ser Thr Tyr Leu Asn Asn Ser Tyr
           195                 200                 205
   Ala Ser Met Pro Gly Thr Ser Met Ala Thr Pro His Val Ala Gly Val
       210                 215                 220
   Ala Ala Leu Val Lys Gln Lys Asn Pro Ser Trp Asn Ala Thr Gln Ile
   225                 230                 235                 240
   Arg Asn His Leu Lys Asn Thr Ala Thr Asn Leu Gly Asn Ser Ser Gln
                   245                 250                 255
   Phe Gly Ser Gly Leu Val Asn Ala Asp Ala Ala Thr Arg
               260                 265
<210> 2
   <211> 269
   <212> PRT
   <213> Bacillus sp.
<400> 2
   Gln Gln Thr Val Pro Trp Gly Ile Thr Arg Val Gln Ala Pro Thr Val
   1               5                   10                  15
   His Asn Arg Gly Val Thr Gly Ser Gly Val Lys Val Ala Ile Leu Asp
               20                  25                  30
   Thr Gly Ile Ala Gln His Ser Asp Leu Thr Ile Arg Gly Gly Ala Ser
           35                  40                  45
   Phe Val Pro Gly Glu Ser Thr Thr Ala Asp Leu Asn Gly His Gly Thr
       50                  55                  60
   His Val Ala Gly Thr Val Ala Ala Leu Asn Asn Ser Ile Gly Val Ile
   65                  70                  75                  80
   Gly Val Ala Pro Ser Ala Asp Leu Tyr Ala Val Lys Val Leu Gly Ala
                   85                  90                  95
   Asn Gly Arg Gly Ser Val Ser Gly Ile Ala Gln Gly Leu Glu Trp Ala
               100                 105                 110
   Ala Thr Asn Asn Met His Ile Ala Asn Met Ser Leu Gly Ser Asp Ala
           115                 120                 125
   Pro Ser Thr Thr Leu Glu Arg Ala Val Asn Tyr Ala Thr Ser Arg Gly
       130                 135                 140
   Val Leu Val Ile Ala Ala Thr Gly Asn Asn Gly Thr Gly Ser Ile Gly
   145                 150                 155                 160
   Tyr Pro Ala Arg Tyr Ala Asn Ala Met Ala Val Gly Ala Thr Asp Gln
                   165                 170                 175
   Asn Asn Arg Arg Ala Ser Phe Ser Gln Tyr Gly Thr Gly Ile Asp Ile
               180                 185                 190
   Val Ala Pro Gly Val Gly Ile Gln Ser Thr Tyr Leu Asn Asn Ser Tyr
           195                 200                 205
   Ala Ser Met Pro Gly Thr Ser Met Ala Thr Pro His Val Ala Gly Val
       210                 215                 220
   Ala Ala Leu Val Lys Gln Lys Asn Pro Ser Trp Asn Ala Thr Gln Ile
   225                 230                 235                 240
   Arg Asn His Leu Lys Asn Thr Ala Thr Asn Leu Gly Asn Ser Ser Gln
                   245                 250                 255
   Phe Gly Ser Gly Leu Val Asn Ala Asp Ala Ala Thr Arg
               260                 265
<210> 3
   <211> 269
   <212> PRT
   <213> Bacillus lentus
<400> 3
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 4
   <211> 810
   <212> DNA
   <213> Bacillus sp.
<400> 4
   caacaaaccg ttccatgggg tattacacgt gtacaagctc ccactgtgca taatcgtgga     60
   gtaacaggat ctggagttaa agtcgctata cttgatacag gtatagctca gcatagtgat    120
   ttaaccattc gtgggggagc aagctttgta ccaggagagt caacaacggc tgatctaaat    180
   ggtcatggta ctcacgttgc tggaacagtg gccgctctta ataattcaat tggtgtgatc    240
   ggtgtggcac caagtgctga cctatacgct gtaaaggtat taggagcaaa tggtagagga    300
   agcgtgagtg ggattgctca aggtctagag tgggctgcaa cgaataacat gcatattgca    360
   aacatgagtc tcggtagtga tgcacctagc actacattag agcgtgcagt taactatgcg    420
   acaagccgtg gagttctcgt cattgcggct actggtaaca atggtactgg ttccattggc    480
   tacccagctc gttatgcaaa cgcaatggct gtaggagcga ctgaccaaaa caacagacgt    540
   gcgagctttt ctcaatatgg cacaggaatt gatattgttg cacctggtgt tggaattcaa    600
   agcacatacc taaataatag ctatgctagt atgcctggaa catcaatggc tacacctcat    660
   gttgctggag tagctgcgct tgttaaacaa aaaaatccat cttggaatgc gactcaaatt    720
   cgtaatcatt tgaaaaatac tgcgacgaat ctaggaaact catctcaatt tggtagtgga    780
   ctagttaatg cagatgcagc aacgcgctaa                                     810